(19) Europäisches Patentamt
European Patent Office
Office européen des brevets

(11) Veröffentlichungsnummer: **0 190 720**
**B1**

(12) # EUROPÄISCHE PATENTSCHRIFT

(45) Veröffentlichungstag der Patentschrift: **02.11.88**

(21) Anmeldenummer: **86101422.3**

(22) Anmeldetag: **05.02.86**

(51) Int. Cl.⁴: **C 07 B 37/04** // C07C15/18, C07C120/00, C07C43/164, C07C69/34, C07C103/34, C07C2/76, C07C41/30, C07C67/00, C07B37/00, C07C2/00, C07C15/00

(54) **Verfahren zur dehydrierenden Dimerisierung und/oder Polymerisierung von organischen Verbindungen mit beweglichen Wasserstoffatomen unter Verwendung von Perketalen.**

(30) Priorität: **06.02.85 DE 3503886**
**24.12.85 DE 3546134**

(43) Veröffentlichungstag der Anmeldung: **13.08.86 Patentblatt 86/33**

(45) Bekanntmachung des Hinweises auf die Patenterteilung: **02.11.88 Patentblatt 88/44**

(84) Benannte Vertragsstaaten: **BE DE FR GB IT NL**

(56) Entgegenhaltungen:
**FR-A-2 200 279**
**US-A-3 009 970**

(73) Patentinhaber: **Luperox GmbH, Wasserburg Denzinger Strasse 7, D-8870 Günzburg (DE)**

(72) Erfinder: **Rauer, Kurt, Dr.Dipl.- Chem., Immelmannstrasse 8, D-8870 Günzburg (DE)**
Erfinder: **Orner- Ziegler, Angelika, Gartenstrasse 5, D-8870 Günzburg (DE)**

(74) Vertreter: **Abitz, Walter, Dr.- Ing., Abitz, Morf, Gritschneder, Freiherr von Wittgenstein Postfach 86 01 09, D-8000 München 86 (DE)**

**EP 0 190 720 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung betrifft ein verbessertes und vereinfachtes Verfahren zur dehydrierenden Dimerisierung oder Polymerisierung von organischen Verbindungen mit beweglichem Wasserstoff an primären, sekundären oder tertiären C-Atomen (z. B. Dimerisierung von Isopropylbenzol zu Biscumyl) mittels Peroxyketale, deren 10 Stunden-Halbwertszeit-Temperaturen unter 115°C liegen (z. B. 1,1-Bis(t-butylperoxy)-cyclohexan), 2,2-Bis(t-butylperoxy)-butan) oder einer Kombination aus Peroxyketalen mit Di-t-butylperoxid unter drucklosen Bedingungen in einer einfachen Apparatur bei Temperaturen zwischen etwa 120°C und 180°C.

Dehydrierende Dimerisationen, Oligomerisationen oder Polymerisationen von organischen Verbindungen mit beweglichem Wasserstoff an C-Atomen werden vorzugsweise mittels Peroxiden, insbesondere Di-t-butylperoxid, ausgeführt. Das typische Beispiel einer solchen dehydrierenden Dimerisierung ist die Umsetzung von Isopropylbenzol mit Di-t-butylperoxid bei Temperaturen oberhalb der Zersetzungstemperatur des Peroxids (also über 100°C) unter Bildung von Biscumyl (= 2,3-Diphenyl-2,3-dimethyl-butan):

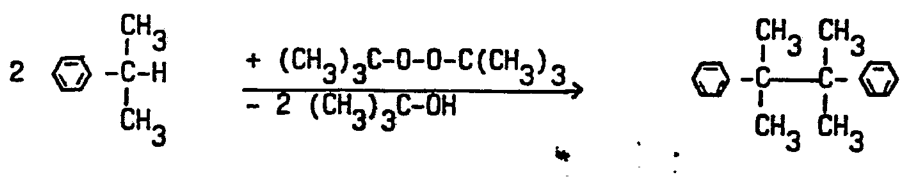


Als Beispiel einer dehydrierenden Poly(oligo)-merisation sei das Erhitzen von Diisopropylbenzol (Gemisch aus m- und p-Isomer) mit Di-t-butylperoxid unter Bildung des polymeren C-C-spaltenden Flammschutzsynergisten (I) genannt:

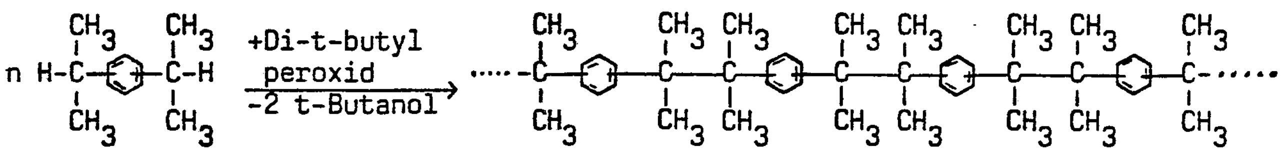


(I)

Neben dem Di-t-butylperoxid wurde als Dehydrierungsmittel für Di(Poly)merisierungen gelegentlich auch Diacetylperoxid verwendet. Beide Peroxide zeichnen sich durch ihr geringes Molekulargewicht aus, das leicht flüchtige Zersetzungsprodukte bedingt, die nach der Oxydation aus dem Reaktionsgemisch durch Destillation leicht zu entfernen sind. Das Diacetylperoxid wäre wegen seiner niedrigen Anspringtemperatur - die 10 Stunden-Halbwertszeit-Temperatur (= 10 hHT) liegt bei 69°C - ein gut brauchbares Dehydrierungsmittel für Dimerisierungen. Wegen seiner Gefährlichkeit kommt es jedoch in Verdünnung als 25 %-iges Produkt in Dimethylphthalat (als eines dar wenigan geeigneten Phlegmatisierungsmittel) in den Handel. Der hohe Siedepunkt des Dimethylphthalats erschwert jedoch die Aufarbeitung des Reaktionsgemisches beträchtlich oder macht sie unmöglich. Das preiswerte und relativ wenig gefährliche Di-t-butylperoxid besitzt den Nachteil einer langen Halbwertszeit (10 hHT = 126°C) verbunden mit einem tiefen Siedepunkt ($Kp_{760}$ = 111°C), so daß oxydative Dimerisierungen mittels Di-t-butylperoxid unter drucklosen Bedingungan einen Zeitraum von 24 - 48 Stdn. erfordern, um annehmbare Ausbeuten zu liefern. Zur Erzielung akzaptebler Reaktionszeiten ist deshalb bei Verwendung von Di-t-butylperoxid als oxydatives Dimerisierungsmittel ein Arbeiten unter Druck im Autoklaven erforderlich. Bei Verwendung von Di-t-butylperoxid liegen die maximalen Ausbeuten an Dimerisierungsprodukt bei etwa 50 - 60 % d. Th. (bezogen auf eingesetztes Peroxid).

Es gibt eine Reihe von Peroxiden mit kürzeren Halbwertszeiten und höheren Siedepunkten als Di-t-butylperoxid, mit deren Hilfe es möglich sein sollte eine oxydative Dimerisierung unter drucklosen Bedingungen in einer wirtschaftlich tragbaren Zeitspanne durchzuführen. Beispiele solcher Peroxide sind Perketale wie 1,1-Bis(t-butylperoxy)-3,3,5-trimethyl-cyclohexan (10 hHT = 92°C), 1,1-Bis(t-butylperoxy)-cyclohexan (10 hHT = 93°C) oder 2,2-Bis(t-butylperoxy)-butan (10 hHT = 100°C), Etherperoxide wie 1-(t-Butylperoxy)-1-methoxy-3,3,5-trimethyl-cyclohexan (10 hHT = ca. 90°C) oder 1-(t-Butylperoxy)-1-methoxy-cyclohexan (10 hHT = ca. 91°C) und Perester wie t-Butylperoxy-pivalat (10 hHT = 55°C), t-Butylperoxy-2-ethylhexanoat (10 hHT = 73°C), t-Butylperoxy-isobutyrat (10 hHT = 79°C) oder t-Butylperoxy-benzoat (10 hHT = 105°C). Der gewünschte hohe Siedepunkt stellt sich allerdings nur am Beginn der dehydrierenden Dimerisierung ein; schon bald danach beobachtet man im Reaktionsgemisch eine schnelle Erniedrigung der Siedetemperatur auf ungefähr 100°C - 110°C infolge Bildung von niedrig siedenden Zersetzungsprodukten obiger Peroxide, insbesondere des t-Butanols, das als Reinprodukt bei 82°C siedet. Eine derart tiefe, bei etwa 100°C - 110°C liegende Reaktionstemperatur bedingt auch bei Peroxiden mit relativ kurzer Halbwertszeit lange Reaktionszeiten um annehmbare Ausbeuten zu erzielen. So wurde eine Lösung von 1,1-Bis(t-butylperoxy)-cyclohexan (10 hHT = 93°C) in überschüssigem Isopropylbenzol unter Rückfluß in einem 150°C heißen Ölbad erhitzt. Die am Anfang der Umsetzung etwa 150°C betragende Innentemperatur der Reaktionsmischung sank

sehr schnell auf 103°C ab. Es wurde die sehr lange Reaktionszeit von 17 Stunden benötigt um eine Ausbeute von 76 % d. Th. an Biscumyl (2,3-Diphenyl-2,3-dimethyl-butan) zu erzielen.

Es wurde jetzt gefunden, daß die Reaktionszeit unter drucklosen Bedingungen bei Verwenden von Bis(alkylperoxy)-ketalen und/oder Bis(aralkylperoxy)-ketalen mit 10 Stunden-Halbwertszeit-Temperaturen unter 115°C als Oxydationsmittel drastisch verkürzt werden kann, wenn das Reaktionsgefäß zusätzlich zum Rückflußkühler einen absteigenden Kühler trägt, der den leicht flüchtigen Zersetzungsprodukten der Peroxide die Möglichkeit gibt abzudestillieren und es damit dem Reaktionsgemisch ermöglicht die viel höhere Temperatur des Ölbads zu erreichen. Auf diese Weise wurde durch Oxydation von Isopropylbenzol mittels 1,1-Bis(t-butylperoxy)-cyclohexan bei einer Ölbadtemperatur von 141°C Biscumyl in einer Ausbeute von 91 % d. Th. nach nur 8 Stunden Reaktionszeit erhalten (gegenüber 76 % d. Th. nach 17 Stunden und 68 % d. Th. nach 8 Stunden unter nichterfindungsgemäßen Bedingungen ohne absteigenden Kühler).

Es wurde gefunden, daß Perketale auf molarer Basis deutlich bessere Ergebnisse, d. h. Ausbeuten, liefern als Etherperoxide, Perester oder Diacylperoxide, welche Ausbeuten von höchstens 50 % gaben, und somit die Verwendung von Perketalen die ökonomischere Lösung darstellt.

Die wirksamsten und preisgünstigsten Perketale sind niedermolekular und werden deshalb aus Sicherheitsgründen in verdünnter Form in Konzentrationen von 75 % bis 50 % gehandhabt. In vielen Fällen kann man die zu di(poly)merisierende Verbindung als Verdünnungsmittel für das Perketal verwenden. Wo dies nicht möglich ist, werden gegen den Angriff von Perketalradikalen inerte, unter 120°C siedende Lösungsmittel, die keinen beweglichen Wasserstoff tragen und vorzugsweise nur $CH_3$-Gruppen und $CH_2$-Gruppen enthalten, verwendet.

Es wurde außerdem gefunden, daß es überraschenderweise trotz des niedrigen Siedepunkt des Di-t-butylperoxids von 111°C möglich ist, die Ausbeute an Di(poly)merisierungsprodukt zu verbessern, wenn zusätzlich zum Perketal (oder zu einem Gemisch von 2 oder mehr Perketalen) Di-t-butylperoxid als zweites Dehydrierungsmittel eingesetzt wird. - Bei Verwendung des Perketals 2,2-Bis(t-butylperoxy)-butan als Dehydrierungsmittel wurden aus Cumol innerhalb einer Gesamtreaktionszeit von 3 Stunden bei einer Ölbadtemperatur von 140° bis 155°C (ansteigend) 93 % d. Th. Biscumyl erhalten. Wurde zusätzlich zum Perketal 2,2-Bis(t-butylperoxy)-butan noch Di-t-butylperoxid eingesetzt und zwar in einer Menge, die 25 Gew.-% der Gesamtmenge des Perketals betrug, das Gew.-%-Verhältnis Perketal zu Di-t-butylperoxid also bei 80 %: 20 % lag, erhöhte sich die Ausbeute an Biscumyl auf 100 % d. Th.. Eine weitere Erhöhung der Di-t-butylperoxid-Menge auf 100 % der Perketalmenge (im Gemisch befanden sich also 50 Gew.-% Perketal und 50 Gew.-% Di-t-butylperoxid) brachte eine Verbesserung der Ausbeute auf 125 % d. Th.. Wurde die Menge an Di-t-butylperoxid noch einmal um das 1,5-fache erhöht, so daß sich im Gemisch 40 Gew.-% Perketal und 60 Gew.-% Di-t-butylperoxid befanden, wurde nur eine geringe weitere Erhöhung der Ausbeute an Biscumyl, nämlich auf 128 % d. Th. erzielt. - Die über 100 % liegenden Ausbeuten erklären sich durch die Art der Ausbeuteberechnung; die Ausbeute wurde nur zur Perketalmenge in Beziehung gesetzt; das Di-t-butylperoxid wurde nicht in die Berechnung einbezogen (wegen der Kostenlosigkeit des aus 2,5-Dimethyl-2,5-bis(t-butylperoxy)-hexin-(3) gewonnenen Di-t-butylperoxids wird es auch in die Berechnung der Materialkosten nicht einbezogen).

Ein auf reguläre Weise produziertes Di-t-butylperoxid ist das billigste aller zur H-Abstraktion fähigen Peroxide. Ein noch preiswerteres Di-tbutylperoxid entsteht als Nebenprodukt bei der Herstellung von t-Butylhydroperoxid üblicherweise in Mengen von ungefähr 15 bis 25 %, un lässt sich auf einfache Weise isolieren un reinigen. Eine preislich noch günstigere Situation liegt im Falle des 2,5-Dimethyl-2,5-bis(t-butylperoxy)-hexin-(3) vor, das im rohen Zustand etwa 30 % Di-t-butylperoxid enthält. Ein handelsfähiges Hexin-Peroxid darf jedoch nur noch maximal 0,5 % Di-t-butylperoxid enthalten. Diese Forderung läßt sich durch Entfernen des Di-t-butylperoxids aus dem Hexin-Peroxid leicht erfüllen. Die Reinheit eines so gewonnenen Di-t-butylperoxids liegt bei höchstens 95 - 96 %, ist deshalb als Handelsprodukt ungeeignet und erfordert somit eine Beseitigung, die Kosten verursacht; in einer Preiskalkulation wird ein solches Di-t-butylperoxid als kostenloser Rohstoff eingesetzt.

Perketale gehören zwar zu den preiswerten Peroxiden, was besonders auf die beiden Perketale 2,2-Bis(t-butylperoxy)-butan und 1,1-Bis(t-butyl-peroxy)-cyclohexan zutrifft, sind aber teurer als Di-t-Butylperoxid und lassen deshalb den Wunsch aufkommen, den Herstellungspreis von Di-(poly)meren, die durch dehydrierende Di(ply)merisierung unter Verwendung von Perketalen als Dehydrierungsmittel erhalten werden, zu senken.

Gegenstand der Erfindung ist demnach ein verbessertes und vereinfachtes Verfahren zur dehydrierenden Dimerisierung und/oder Polymerisierung von organsichen Verbindungen mit beweglichem Wasserstoff an primären, sekundären oder teritären C-Atomen, die bie Atmosphärendruck bei mindestens 95°C sieden und keine Gruppen enthalten, unter Verwendung von Peroxiden als Dehydrierungsmittel und unter drucklosen Bedingungen bei Temperaturen von unterhalb bis knapp oberhalb des Siedepunktes der zu dehydrierenden Verbindungen, aber oberhalb der Zersetzungstempertur der als Dehydrierungsmittel wirkenden Peroxide, in einer Vorrichtung die ein beheizbares Reaktionsgefäß und einen damit verbundenen Rückflußkühler aufweist, wobei man

- als dehydrierende Peroxide ein oder mehrere Bis(alkylperoxy)-ketale und/oder Bis(aralkylperoxy)-ketale mit 10 Stunden-Halbwertszeit-Temperaturen von weniger als 115°C (bestimmt in Benzol) oder eine Kombination aus einem oder mehreren dieser Perketale mit Di-t-butylperoxid verwendet, und
- die flüchtigen Peroxidzersetzungsprodukte und gegebenenfalls einen Teil des Di-t-butylperoxids

durch einen, zusätzlich zum Rückflußkühler vorhandenen, absteigenden Kühler abdestilliert.

Zum Beispiel leigt die Verfahrenstemperatur zwischen 100° und 180°C, vorzugsweise bei wenigstens 120°C, insbesondere zwischen 130° und 155°C. Die Zugabe des Peroxids kann durch den Rückflußkühler erfolgen, vorzugsweise durch ein Gasrohr, das von oben durch den Rückflußkühler bis knapp unter die Oberfläche des Reaktionsgemisches reicht.

Dem absteigenden Kühler kann gegebenenfalls eine Destillationskolonne vorgeschaltet sein. Das mit dem Reaktionsgefäß verbundene Ende des absteigenden Kühlers darf sich daher - im Gegensatz zum Rückflußkühler, dessen unteres Ende in jeder beliebigen Höhe über dem Reaktionsgefäß beginnen darf - nur so hoch über dem Reaktionsgefäß befinden, daß ein Überdestillieren der flüchtigen Peroxidzersetzungsprodukte und gegebenenfalls des Di-t-butylperoxids nicht verhindert wird.

Das Verfahren wird gegebenenfalls in Gegenwart von geeigneten, gegen den Angriff von Peroxidradikalen inerten, unter 120°C siedenden Lösungsmittel, die kleinen beweglichen Wasserstoff tragen und vorzugsweise nur $CH_3$-Gruppen und $CH_2$-Gruppen enthalten, vorgenommen.

Das erfindungsgemäße, dehydrierende Dimerisierungs- und Polymerisierungsverfahren hat folgende Vorteile:

- Das erfindungsgemäße, drucklose Dehydrierungsverfahren erfordert nur eine einfache Apparatur mit - verglichen mit einem Autoklaven - erheblich geringeren Investitionskosten.
- Im Autoklavenverfahren wird die gesamte Peroxidmenge der zu oxydierenden Verbindung vor Beginn der Umsetzung zugesetzt. Ein derartiges, eingeschlossenes Peroxid enthaltendes Gemisch stellt beim Erhitzen eine erhebliche Gefahr dar. Dagegen ermöglicht und empfiehlt das erfindungsgemäße, druchlose Verfahren die allmähnliche Zugabe des Peroxids zum heißen Reaktionsgemisch, dessen Temperatur oberhalb der Zersetzungstemperatur des Peroxids liegt und deshalb im Reaktionsgemisch immer nur in kleiner Menge vorliegt.
- Im Vergleich zu einem drucklosen Dehydrierungsverfahren ohne absteigenden Kühler kommt das erfindungsgemäße Verfahren mit absteigenden Kühler mit erheblich verkürzten Reaktionszeiten aus und es liefert außerdem deutlich höhere Ausbeuten.

In einer typischen Ausführungsform des erfindungsgemäßen Dehydrierungsverfahrens wird ein kleinerer Teil der zu oxydierenden Verbindung in den Reaktor vorgelegt. Der größere Rest der zu oxydierenden Verbindung wird mit der Gesamtmenge des bzw. der Peroxide vermischt und diese Peroxidlösung wird durch ein Rohr, das durch den Rückflußkühler geführt wird und in die auf Reaktionstemperatur aufgeheizte gerührte Flüssigkeit eintaucht, allmählich zugetropft. Während dieser Zeit destillieren die flüchtigen Zersetzungsprodukte der Peroxide und, sofern vorhanden, auch ein Teil des Di-t- butylperoxids durch den absteigenden Kühler ab. Die Ölbadtemperatur (bzw. die Temperatur der Heizflüssigkeit der Reaktionsmantelheizung) liegt vorzugsweise bei 130° - 155°C. Nach beendeter Zugabe des Peroxids wird das Reaktionsgemisch noch einmal etwa die gleiche Zeitspanne auf der Reaktionstemperatur oder auf einer höheren Temperatur gehalten, wobei die erhöhte Temperatur in einer oder mehreren Stufen erreicht wird. Danach destilliert man den überschüssigen Anteil der zu di(poly)merisierenden Verbindung unter Atmosphärendruck oder im Vakuum ab und arbeitet den Rückstand - z. B. durch Umkristallisieren aus Alkohol - auf. Die zu oxydierende Verbindung wird in bezug auf das Peroxid-Gemisch in beliebigem molaren Überschuß eingesetzt, mindestens aber 2 Mol zu oxydierende Verbindung auf 1 Mol Peroxidgemisch.

Während des Zutropfens des Peroxidgemisches aus Perketal und Di-t- butylperoxid und während der Nachreaktion destilliert ein beträchtlicher Teil des Di-t-butylperoxids gemeinsam mit den niedrigsiedenden Peroxidzersetzungsprodukten, hauptsächlich t-Butanol, ab. Ein anderer Teil des Di- t-butylperoxids zersetzt sich im Reaktionsgemisch unter Bildung von t-Butoxy-Radikalen, $(CH_3)_3C$-O, welche die erwünschte Dehydrierungsreaktion bewirken. Der dritte Teil des Di-t-butylperoxids verbleibt, wegen seiner langen Halbwertszeit, unzersetzt im Reaktionsgemisch. Dieser Anteil des Di- t-butylperoxids geht beim Destillieren der unverbrauchten, zu dehydrierenden Verbindung mit dieser über. Die beiden Anteile Di-t-butylperoxid, die sich in den Destillaten befinden, lassen sich für den nächsten Ansatz der gleichen Reaktion wiederverwenden: das während der Reaktion erhaltene, hauptsächlich t-Butanol und Di-t-butylperoxid enthaltende Destillat läßt sich mit zwei bis drei Wasserwäschen vom t-Butanol befreien; nach Trocknen mit z. B. $Na_2SO_4$ oder $MgSO_4$ ist das Di-t-butylperoxid bereit für den nächsten Ansatz. Das in der durch Destillation wiedergewonnenen, unverbrauchten, zu dehydrierenden Verbindung befindliche Di-t-butylperoxid läßt sich gemeinsam mit der zu dehydrierenden Verbindung für den nächsten Ansatz wiederverwenden, gegebenenfalls nach Wasserwäsche(n) oder alkalischer Wäsche(n) und Trocknung. - In einem Ansatz zur Herstellung von Biscumyl aus 146 g Cumol durch Umsetzung mit einem Gemisch aus gleichen Gewichtsteilen des Perketals 2,2-Bis(t-butylperoxy)-butan (34,2 g) und Di-t-butylperoxid (34,2 g) wurden im t-Butanol enthaltenden 32,6 g "Reaktions"-Destillat 25,7 % (= 8,4 g) Di-t-butylperoxid und im Cumol-Destillat (91,7 g) 7,1 % (= 6,5 g) Di-t-butylperoxid gaschromatographisch nachgewiesen, das sind 43,6 % des eingesetzten Di-t-butylperoxids, die wiederverwendbar sind.

Während des Destillierens befindet sich im Gasraum Di-t-butylperoxid. Dieses läßt sich im reinen Zustand gefahrlos destillieren. In beiden Destillationen ist das Di-t-butylperoxid im Gasraum mit t-Butanol bzw. der zu dehydrierenden Verbindung vermischt und damit phlegmatisiert. Trotzdem empfiehlt es sich zur zusätzlichen

Phlegmatisierung, einen langsamen Stickstoffstrom oder Kohlendioxydstrom während des Destillierens durch die Apparatur zu leiten.

Wie bereits oben erwähnt, wird Di-t-butylperoxid bei der Reinigung des 2,5-Dimethyl-2,5-bis(t-butylperoxy)-hexin-(3) als ansonsten nicht verwertbares Abfallprodukt gewonnen. Es wird daher nicht in die Berechnung der Materialkosten des Di(poly)meren einbezogen. In einem Ansatz zur Herstellung von Biscumyl aus 146 g Cumol und 34,2 g des Perketals 2,2-Bis(t-butylperoxy)-butan lagen die Materialkosten daher um 37 % höher als bei Verwendung eines Gemisches aus 34,2 g 2,2-Bis(t-butylperoxy)-butan und 34,2 g Di-t-butylperoxid. Im entsprechenden Versuch mit 1,1-Bis(t-butylperoxy)-cyclohexan lagen die Materialkosten sogar um 38,5 % höher.

Die dimerisierende bzw. polymerisierende Wirkung von Peroxiden, in der vorliegenden Erfindung von Perketalen und gegebenenfalls Di-t-butylperoxid, beruht auf ihrer Fähigkeit zur H-Abstraktion von aktivierten, also lockerer gebundenen, Wasserstoffatomen unter Bildung von Radikalen, die - je nach Struktur der organischen Verbindung - dimerisieren oder polymerisieren. So reagiert beispielsweise Isopropylbenzol (= Cumol) mit einem aus einem Perketal oder Di-t-butylperoxid stammenden t-Butoxy-Radikal unter H-Abstraktion zum Cumyl-Radikal, das zum Biscumyl dimerisiert:

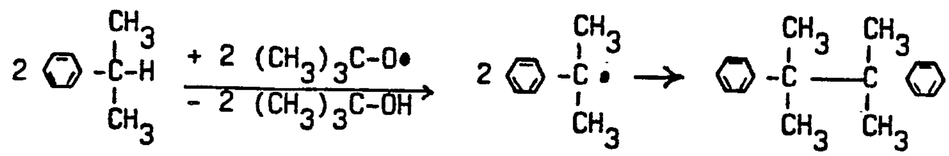


(Cumol) (Cumyl-Radikal) (Biscumyl)

Als Beispiele für dehydrierend zu di- oder polymerisierende Verbindungen mit beweglichem Wasserstoff an primären, sekundären oder tertiären C-Atomen seien genannt:

(a) Verbindungen mit Wasserstoff an primären C-Atomen: N-Methyl-acetamid, N,N-Dimethylacetamid, Tetramethyl-harnstoff, N,N-Dimethyl-isobutyramid, N,N-Dimethyl-benzolsulfonamid, Toluol, Xylol.

(b) Verbindungen mit Wasserstoff an sekundären C-Atomen: Malonsäuredimethylester, 2-Methoxyessigsäure-methylester, 1,2,3-Trioxan, Ethylbenzol, Diphenylmethan.

(c) Verbindungen mit Wasserstoff an tertiären C-Atomen: Isopropyl-benzol (= Cumol), sec.Butyl-benzol, o- oder m- oder p-Diisopropyl-benzol, N-Butyl-formamid, 1-Phenyl-1-methoxy-ethan, 1,1-Diphenyl-dimethylether, N-Isopropylsuccinimid, N-Isopropylphthalimid, 2-Methyl-malonsäuredimethylester oder -diethylester, 2-Ethyl-malonsäuredimethylester oder -diethylester, Isobuttersäure-n-butyl-ester, 2-Phenyl-2-methyl-essigsäuremethylester oder -ethylester, 1,1,2-Trimethoxyethan.

Nicht geeignet für das vorliegende erfindungsgemäße Dimerisierungs- bzw. Polymerisierungsverfahren sind Verbindungen, die bei Atmosphärendruck unterhalb 95°C sieden, da wegen der tiefen Reaktionstemperatur, die Di-(Poly)merisierungszeiten unwirtschaftlich lang werden, aber auch Verbindungen mit freien Carbonsäuregruppen oder anderen sauren Gruppen, wegen der Säureempfindlichkeit von Perketalen.

Bevorzugt für das erfindungsgemäße Dimerisierungs- bzw. Polymerisierungsverfahren sind Verbindungen mit beweglichem Wasserstoff an tertiären C-Atomen (z. B. Isopropylbenzol), aus denen vorzugsweise C-C-spaltende Initiatoren, Vernetzungsmittel oder Flammschutzsynergisten entstehen.

Beispiele für die als Oxydationsmittel verwendeten Perketale sind

a) Perketale aus Monoketonen (2 Peroxid-Gruppen enthaltend):
2,2-Bis(t-butylperoxy)-propan (10 hHT = 105°C), 2,2-Bis(t-butylperoxy)-butan (10 hHT = 100°C), 2,2-Bis(t-butylperoxy)-4-methyl-pentan (10 hHT = 100°C); 3,3-Bis(t-butylperoxy)-buttersäure-ethylester (10 hHT = 113°C), 4,4-Bis(t-butylperoxy)-lävulinsäure-n-butylester (10 hHT = 109°C), 5,5-Bis(t-butylperoxy)-hexansäure-ethyl (oder n-butyl)ester (10 hHT = 106°C), 1,1-Bis(t-butylperoxy)-cyclohexan (10 hHT = 93°C), 1,1-Bis(t-butylperoxy)-3,3,5-trimethylcyclohexan (10 hHT = 92°C), 1,1-Bis(t-butylperoxy)-1-phenyl-ethan, 4,4-Bis(t-butylperoxy)-2,2,6,6-tetramethylpiperidin, 4,4-Bis(t-butylperoxy)-2,2,6,6-tetramethyl-piperidinium-acetat, sowie die analogen Perketale, in denen t-Butylperoxy- durch anderes Hydroperoxy- wie t-Amylperoxy-, t-Octylperoxy-(=1,1,3,3-Tetramethyl-butyl-peroxy-) oder Cumylperoxy- ersetzt ist.

b) perketale aus Diketonen (4 Peroxid-Gruppen enthaltend):
Butandiol-(1,4)-bis(3,3-bis(t-butylperoxy)-butyrat), Hexandiol-(1,6)-bis(3,3-bis(t-butylperoxy)-butyrat) Diethylenglykol-bis(3,3-bis(t-butylperoxy)-butyrat) sowie die analogen Perketale, in denen t-Butylperoxy- durch anderes Hydroperoxy- wie t-Amylperoxy-, t-Octylperoxy- oder Cumylperoxy ersetzt ist.

c) Perketale aus Monoketonen und Dihydroperoxiden (4 Peroxid-Gruppen enthaltend):

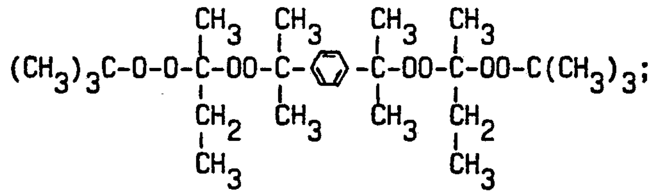
;

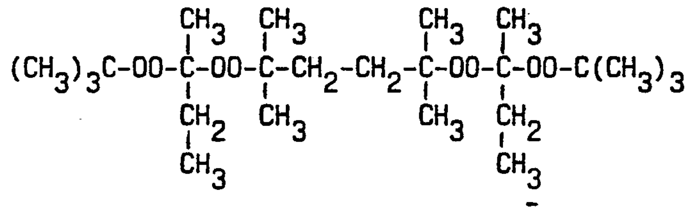

sowie die analogen Perketale, in denen Methylethylketon als Ausgangsketon durch andere Ketone wie Aceton, Methylisobutylketon, Acetessigester, Lävulinsäure-n-butylester, 5-Ketohexansäure-ethylester, Cyclohexanon, 3,3,5-Tri-methylcyclohexanon, Acetophenon ersetzt wurde, und außerdem solche Perketale, in denen t-Butylperoxy durch anderes Hydroperoxy wie t-Amylperoxy-, t-Octylperoxy- oder Cumylperoxy ersetzt ist.

Beispiele für inerte, zum Verdünnen der Perketale geeignete Lösungsmittel sind n-Pentan, n-Hexan, n-Heptan, n-Octan, Cyclohexan, Benzol (wegen seiner carcinogenen Eigenschaften für die industrielle Praxis nicht empfehlenswert), Tetramethyl-methan, Methyl-t-butyl-ether. - Als wenig geeignet haben sich z. B. n-Decan, Toluol oder Xylol erwiesen.

Die Erfindung wird durch die nachstehenden Beispiele erläutert.

**BEISPIELE**

**Beispiel 1:**

Typisches erfindungsgemäßes Beispiel zur Herstellung von Biscumyl (=2,3-Diphenyl-2,3-dimethyl-butan) aus Cumol

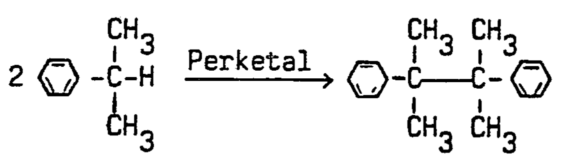


Mol.-Gew. = 238,36
Schmp. (rein) = 119° - 120°C

Mol.-Gew. = 238,36
Schmp. (rein) = 119° - 120°C

unter Verwendung des Perketals 1,1-Bis(t-butylperoxy)-cyclohexan:

32 g Cumol (= Isopropylbenzol) (0,266 Mol) wurden in einem 250 ml-Dreihalsrundkolben eingewogen, der mit einem Rückflußkühler (am Mittelhals), einem absteigenden Kühler (an einem der zwei Außenhälse) und einem in das eingewogene Cumol hineinreichenden Thermometer (am zweiten der zwei Außenhälse) verbunden ist. Außerdem wurde durch den Rückflußkühler ein Glasrohr gesteckt, dessen unteres Ende unter die Oberfläche des Reaktionsgemisches reichte. Während des Aufheizens und der Umsetzung wurde mit einem Magnetrührer gerührt. Der Rundkolben befand sich in einem Ölbad. Man erwärmte das Ölbad auf 132°C und ließ - sobald das im Kolben befindliche Cumol eine Temperatur von etwa 130°C erreicht hatte - innerhalb 4 Stunden unter Rühren durch das durch den Rückflußkühler reichende, in das Cumol eintauchende Glasrohr eine Lösung von 38 g 1,1-Bis(t-butylperoxy)-cyclohexan (0,146 Mol) in 80 g Cumol (0,665 Mol) zutropfen. Da während der Umsetzung die flüchtigen Zersetzungsprodukte des Perketals abdestillieren, stellte sich bei einer Ölbadtemperatur von 132°C eine Innentemperatur von 123°C ein. Man ließ 4 Stunden unter Rühren nachreagieren, wobei während der ersten 1,85 Stunden der Nachrührzeit die ursprüngliche Ölbadtemperatur von 132°C aufrecht erhalten wurde, die Ölbadtemperatur innerhalb 9 Minuten auf 141°C erhöht wurde und die letzten 2 Stunden bei einer Ölbadtemperatur von 141°C gerührt wurde, wobei sich eine Innentemperatur von 129°C einstellte. Die Gesamtreaktionszeit betrug also 8 Stunden. Danach wurde das überschüssige Cumol

(Siedepunkt: 152° - 154°C) im Wasserstrahlvakuum bei maximal 100°C Ölbadtemperatur abdestilliert. Der Rückstand wurde aus 200 ml Isopropanol umkristallisiert und gab nach Absaugen und Trocknen an der Luft bei Raumtemperatur 22,7 g Biscumyl als weiße, kristalline Substanz vom Schmelzpunkt 114° - 119°C.

Durch vollständiges Abdestillieren des Isopropanols aus dem Filtrat und Umkristallisieren des Rückstandes aus 10 ml Isopropanol wurden weitere 8,8 g weißes kristallines Biscumyl vom Schmelzpunkt 108° - 111°C erhalten. Die Gesamtausbeute an Biscumyl liegt demnach bei 31,5 g (91 % d. Th.). Sowohl das abdestillierte Cumol als auch das abdestillierte Isopropanol lassen sich wiederverwenden.

**Vergleichsbeispiel A:**

Dieses Beispiel beschreibt die Herstellung von Biscumyl aus Cumol unter Verwendung des Perketals 1,1-Bis(t-butylperoxy)-cyclohexan unter nichterfindungsgemäßen Bedingungen mit entsprechend schlechteren Ausbeuten.

(a) 142 g Cumol (1,18 Mol) und 38 g 1,1-Bis(t-butylperoxy)-cyclohexan (0,146 Mol) wurden wie in Beispiel 1 zur Reaktion gebracht, jedoch ohne Verwendung eines absteigenden Kühlers, weshalb die Temperatur des Reaktionsgemisches während der gesamten Reaktionszeit von 8 Stunden bei 110°-112°C lag (mit Ausnahme der ersten 15 Minuten, in denen die Reaktionstemperatur von anfangs 123°C allmählich auf 110° - 112°C absank). Diese tiefe Reaktionstemperatur von 110° - 112°C erklärt sich aus der Bildung von leicht flüchtigen Zersetzungsprodukten aus dem Perketal, z. B. von t-Butanol (Siedepunkt: 82°C), die infolge Fehlens des absteigenden Kühlers aus dem Reaktionsgemisch nicht abdestillieren können. Man erhält so 17,5 g Biscumyl vom Schmp. 111° - 117°C und aus der Mutterlauge 5,3 g Biscumyl vom Schmp. 106° - 111°C; insgesamt also nur 22,8 g (66 % d. Th.) Biscumyl (in Form weißer Kristalle).

(b) Wie oben unter (a) ebenfalls ohne absteigenden Kühler, jedoch bei einer Ölbadtemperatur von 140° - 150°C während der gesamten Reaktionszeit, einer Zutropfzeit des Perketals von 5 Stunden und einer Nachreaktionszeit von 12 Stdn., also einer Gesamtreaktionszeit von 17 Stunden, wobei die Reaktionstemperatur während der gesamten 17 Stdn. bei 103°C lag (mit Ausnahme der ersten etwa 15 Minuten, in der sie von 123° auf 103°C fiel). Auf diese Weise wurden 21,2 g Biscumyl vom Schmp. 115° - 118°C und aus der Mutterlauge 5,3 g Biscumyl vom Schmp. 107° - 116°C, insgesamt also nur 26,5 g (76 % d. Th.) Biscumyl (in Form weißer Kristalle) trotz der langen Reaktionszeit von 17 Stunden erhalten.

**Vergleichsbeispiel B:**

Dieses Beispiel beschreibt die Herstellung von Biscumyl aus Cumol unter ebenfalls nichterfindungsgemäßen Bedingungen, nämlich unter Verwendung von Di-t-butylperoxid:

(a) 142 g Cumol (1,18 Mol) und 21,8 g Di-t-butylperoxid, 98 %-ig (0,146 Mol) wurden wie in Beispiel 1 zur Reaktion gebracht (wurden also 8 Stdn. mit absteigendem Kühler erhitzt). Auf diese Weise wurden 8,9 g Biscumyl vom Schmp. 116° - 119°C und aus der Mutterlauge 0,9 g Biscumyl vom Schmp. 111° - 115°C, insgesamt also nur 9,8 g (28 % d. Th.) Biscumyl erhalten. - - (Der analoge Versuch unter Verwendung des erfindungsgemäßen Perketals 1,1-Bis(t-butylperoxy)-cyclohexan gab eine Ausbeute an Biscumyl von 91 % d. Th. (Beispiel 1)).

(b) 142 g Cumol (1,18 Mol) und 21,8 g Di-t-butylperoxid, 98 %-gi (0,146 Mol) wurden wie in Beispiel 1 zur Reaktion gebracht, jedoch ohne Verwendung eines absteigenden Kühlers und bei einer Ölbadtemperatur von 140°-150°C, einer Zutropfzeit des Peroxids von 5 Stunden und einer Nachreaktionszeit von 12 Stunden, also einer Gesamtreaktionszeit von 17 Stunden, wobei die Innentemperatur von anfangs 135°C bald auf 114°C absank. Auf diese Weise wurden 10 g Biscumyl vom Schmp. 116° - 119°C und aus der Mutterlauge 0,4 g Biscumyl vom Schmp. 112° - 115°C, insgesamt also nur 10,4 g (30 % d. Th.) Biscumyl erhalten. -- (Der analoge Versuch unter Verwendung des Perketals 1,1-Bis(t-butylperoxy)-cyclohexan gab eine Ausbeute von 76 % d. Th. Biscumyl (Vergleichsbeispiel A (b)).

(c) Wie oben unter (b), also ebenfalls ohne absteigenden Kühler, aber einer Zutropfzeit von 4 Stunden und einer Nachreaktionszeit von 6 Stunden, also einer Gesamtreaktionszeit von 10 Stunden. Auf diese Weise wurden 8,1 g Biscumyl vom Schmp. 116° - 120°C und aus der Mutterlauge 0,6 g Biscumyl vom Schmp. 114° - 117°C, insgesamt also nur 8,7 g (25 % d. Th.) Biscumyl erhalten.

**Beispiel 2:**

In diesem erfindungsgemäßen Beispiel wird auf die gleiche Weise wie in Beispiel 1 Biscumyl aus Cumol unter Verwendung von 38 g 1,1-Bis(t-butylperoxy)-cyclohexan (0,146 Mol) hergestellt, jedoch mit einer Zutropfzeit von 3 Stunden, also einer Gesamtreaktionszeit von nur 6 Stunden (anstelle von 8 Stunden im Beispiel 1). Man erhielt so 20,9 g Biscumyl vom Schmp. 110°-116°C und aus der Mutterlauge 7,1 g Biscumyl vom Schmp 106° - 111°C, insgesamt also einer Gesamtausbeute von 28 g (81 % d. Th.). -- (Im Beispiel 1 wurden nach 8 Stunden Gesamtreaktionszeit 91 % d. Th. Biscumyl erhalten).

**Beispiel 3:**

In diesem erfindungsgemäßen Beispiel wurde Biscumyl wie in Beispiel 1 aus Cumol, jedoch unter Verwendung von 0,146 Mol der Perketale 3,3-Bis(t-butylperoxy)-buttersäure-ethylester (= Lup.233), 4,4-Bis(t-butylperoxy)- valeriansäure-n-butylester (= Lup.230), 2,2-Bis(t-butylperoxy)-butan (= Lup.220) und 1,1-Bis(t-butylperoxy)-3,3,5-trimethyl-cyclohexan (= Lup.231) mit folgenden guten Ergebnisse hergestellt:

| Perketale | Biscumyl aus 1. Umkristallisation; Schmp. | Biscumyl aus Mutterlauge; Schmp. | Gesamtausbeute in g; in % d. Th. |
|---|---|---|---|
| Lup.233 | 13,2 g<br>112° - 117°C | 11 7 g<br>109° - 115°C | 24,9 g<br>72 % d. Th. |
| Lup.230 | 14,2 g<br>112° - 117°C | 10,7 g<br>110° - 115°C | 24,9 g<br>72 % d. Th. |
| Lup.220 | 31,2 g<br>113° - 117°C | 3,5 g<br>105° - 112°C | 34,7 g<br>100 % d. Th. |
| Lup.231 | 21,2 g<br>112° - 116°C | 1,9 g<br>111° - 116°C | 23,1 g<br>67 % d. Th. |

**Vergleichsbeispiel C:**

In diesem Beispiel wurde Biscumyl wie in Beispiel 1 aus Cumol, jedoch unter Verwendung von 0,146 Mol der nichterfindungsgemäßen Perester t-Butylperoxy-benzoat (= Lup.P), t-Butylperoxy-isobutyrat, (= Lup.80), t-Butylperoxy-2-ethyl-hexanoat (= Lup.26-R), und t-Butylperoxy-pivalat (= Lup.11), mit folgenden Ausbeuten hergestellt, die deutlich unter denen der erfindungsgemäßen Perketale aus den Beispielen 1 und 3 liegen.

| Perester | Biscumyl aus 1. Umkristallisation; Schmp. | Biscumyl aus Mutterlauge; Schmp. | Gesamtausbeute in g; in % d. Th. |
|---|---|---|---|
| Lup.11 | 7,3 g<br>114° - 118°C | 3,1 g<br>107° - 113° | 10,4 g<br>30 % d. Th. |
| Lup.80 | 11,1 g<br>114° - 118°C | 2,6 g<br>109° - 115°C | 13,7 g<br>40 % d. Th. |
| Lup.26-R | 9,2 g<br>114° - 118°C | 2 9 g<br>111° - 117°C | 12,1 g<br>35 % d. Th. |
| Lup.P | 11,2 g<br>112° - 117°C | 4,4 g<br>108° - 113°C | 15,6 g<br>45 % d. Th. |

**Vergleichsbeispiel D:**

In diesem Beispiel wurde Biscumyl wie in Beispiel 1 aus Cumol, jedoch unter Verwendung von 0,146 Mol der nichterfindungsgemäßen Etherperoxide 1-t-Butylperoxy-1-methoxy-cyclohexan und 1-t-Butylperoxy-1-

methoxy-3,3,5-trimethyl-cyclohexan, mit folgenden Ausbeutan hergestellt, die deutlich unter denen der erfindungsgemäßen Perketale aus den Beispielen 1 und 3 liegen:

| Etherperoxide | Biscumyl aus 1. Umkristallisation; Schmp. | Biscumyl aus Mutterlauge; Schmp. | Gesamtausbeute in g; in % d. Th. |
|---|---|---|---|
| 1-t-Butylperoxy-1-methoxy-3,3,5-tri-methyl-cyclohexan | 6,3 g<br>116° - 119°C | 2,7<br>114° - 118°C | g 9 g<br>26 % d. Th. |
| 1-t-Butylperoxy-1-methoxy-cyclohexan | 12,2 g<br>115° - 118°C | 4,3 g<br>110° - 115°C | 16,5 g<br>48 % d. Th. |

**Vergleichsbeispiel E:**

In diesem Beispiel wurde Biscumyl wie in Beispiel 1 aus Cumol, jedoch unter Verwendung von 0,146 Mol der nichterfindungsgemäßen Diacylperoxide Didecanoylperoxid und Dibenzoylperoxid, mit folgenden Ausbeuten hergestellt, die deutlich unter denen der erfindungsgemäßen Perketale aus den Beispielen 1 und 3 liegen (wegen der geringen Löslichkeit von Dibenzoylperoxid in Cumol mußte die Cumolmenge von 112 g auf 532 g erhöht werden):

| Diacylperoxid | Biscumyl aus 1. Umkristallisation; Schmp. | Biscumyl aus Mutterlauge; Schmp. | Gesamtausbeute in g; in % d. Th. |
|---|---|---|---|
| Didecanoylperoxid | 7,25 g<br>113° - 116°C | 0,75 g<br>100° - 108°C | 8,0 g<br>23 % d. Th. |
| Dibenzoylperoxid* | 7,5 g<br>115° - 117°C | 0,5 g<br>111° - 115°C | 8,0 g<br>23 % d. Th. |

* Nach beendeter Reaktion und nach dem Abkühlen war das Reaktionsgemisch zur Entfernung der Benzoesäure zweimal mit 400 ml 5 %-iger Kalilauge und einmal mit 400 ml Wasser gewaschen und anschließend mit 5 g $MgSO_4$ getrocknet worden.

**Beispiel 4:**

In diesem erfindungsgemäßen Beispiel wurde Biscumyl wie in Beispiel 1 aus Cumol unter Verwendung von 38 g des Perketals 1,1-Bis(t-butylperoxy)-cyclohexan (0,146 Mol) hergestellt; die mit Cumol verdünnte Perketallösung enthält jedoch zusätzlich 25 g der in der nachstehenden Tabelle genannten Lösungsmittel. Aus der nachstehenden Tabelle ist zu erkennen, daß alle geprüften Lösungsmittel einen erkennbaren negativen Einfluß auf die Ausbeuten haben. -- (Ohne zusätzliches Lösungsmittel betrug die Ausbeute (in Beispiel 1) 91 % d. Th.).

| Lösungsmittel | Biscumyl aus 1. Umkristallisation; Schmp. | Biscumyl aus Mutterlauge; Schmp. | Gesamtausbeute in g; in % d. Th. |
|---|---|---|---|
| n-Pentan | 20,2 g<br>115° - 117°C | 2,4 g<br>110° - 115°C | 22,6 g<br>65 % d. Th. |
| t-Butyl-methyl-ether | 21,8 g<br>115° - 118°C | 1,7 g<br>105° - 113°C | 23,5 g<br>68 % d. Th. |
| n-Hexan | 22,0 g<br>115° - 118°C | 0,6 g<br>107° - 113°C | 22,6 g<br>65 % d. Th. |
| Cyclohexan | 21,7 g<br>114° - 118°C | 1,4 g<br>103° - 112°C | 23,1 g<br>67 % d. Th. |
| n-Heptan | 20,1 g<br>113° - 116°C | 1,3 g<br>105° - 112°C | 21,4 g<br>62 % d. Th. |
| n-Decan | 20,1 g<br>115° - 118°C | 1,1 g<br>100° - 109°C | 21,2 g<br>61 % d. Th. |
| Toluol | 17,7 g<br>113° - 116°C | 2,3 g<br>102° - 111°C | 20,0 g<br>58 % d. Th. |
| Xylol | 14,9 g<br>113° - 117°C | 0,9 g<br>102° - 113°C | 15,8 g<br>45,5 % d. Th. |

**Beispiel 5:**

In diesem Beispiel wird eine Verbindung mit einem sekundären C-Atom, nämlich der Malonsäuredimethylester durch Umsetzung mit dem Perketal 1,1-Bis(t-butylperoxy)-cyclohexan analog Beispiel 1 zum Ethan-1,1,2,2-tetra-carbonsäure-tetramethylester dimerisiert:

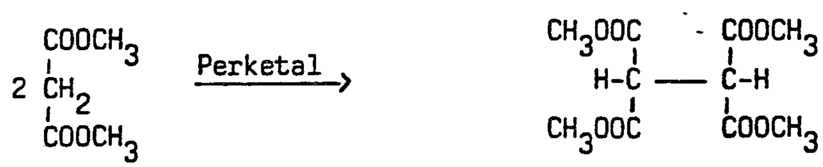


Abweichend von Beispiel 1 wurde das Perketal als 75,3 %-ige Lösung in n-Pentan eingesetzt; somit befanden sich vor Beginn der Umsetzung im Rundkolben 35,1 g Malonsäuredimethylester (0,266 Mol) und im Tropftrichter eine Lösung von 38 g 1,1-Bis(t-butylperoxy)-cyclohexan (0,146 Mol) in 12,5 g n-Pentan und 87,9 g Malonsäuredimethylester (0,665 Mol). Bei der Ölbadtemperatur von 132°C lag die Innentemperatur bei 127° - 128°C und bei der erhöhten Ölbadtemperatur von 141°C lag die Innentemperatur bei 137°C. Nach dem Abdestillieren des unumgesetzten Malonsäuredimethylesters i. Vak. bei etwa 120°C wurden zum Rückstand 5 ml Methanol gegeben. Nach Stehen über Nacht wurde der ausgefallene Ethan-1,1,2,2-tetracarbonsäure-tetramethylester abgesaugt und mit wenig Methanol nachgewaschen; weiße nadelförmige Kristalle; Ausbeute: 1,7 g (4,4 % d. Th.); Schmp: 131° - 133°C (Literatur: 136° - 138°C).

**Beispiel 6:**

In diesem Beispiel wird eine stickstoffhaltige Verbindung mit einem tertiären C-Atom, nämlich N-t-Butyl-formamid, durch Umsetzung mit dem Perketal 1,1-Bis(t-butylperoxy)-cyclohexan analog Beispiel 1 zum oxalsäure-bis(t-butylamid) dimerisiert:

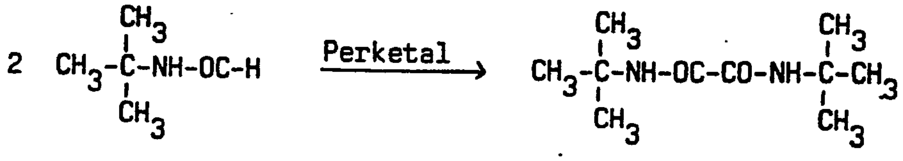


Abweichend von Beispiel 1 wurde das Perketal als 75,3 %-ige Lösung in n-Pentan eingesetzt; somit befanden sich vor Beginn der Umsetzung im Rundkolben 26,9 g N-t-Butyl-formamid (0,266 Mol) und im Tropftrichter eine Lösung von 38 g 1,1-Bis(t-butylperoxy)-cyclohexan (0,146 Mol) in 12,5 g n-Pentan und 67,3 g N-t-Butylformamid (0,665 Mol). Bei der Ölbadtemperatur von 132°C lag die Innentemperatur bei 127° - 128°C und bei der erhöhten Ölbadtemperatur von 141°C lag die Innentemperatur bei 135°C. Nach dem Abdestillieran des unumgesetzten N-t-Butyl-formamids i. Vak. bei etwa 120°C wurden zum abgekühlten Rückstand etwa 20 ml Methanol gegeben. Nach längerem Stehen bei Raumtemperatur bildeten sich Kristalle, die abgesaugt und mit Methanol nachgewaschen wurden; man erhielt so 0,1 g Oxalsäure-bis(t-butylamid) als weiße Kristalle vom Schmp. 167° - 171°C. Im Destillat, welches das überschüssige N-t-Butyl-formamid enthielt, bildeten sich nach längerem Stehen ebenfalls Kristalle, die abgesaugt und mit Methanol nachgewaschen wurden; man erhielt so 0,5 g Oxalsäure-bis(t-butylamid) als weiße Kristalle vom Schmp. 172° - 174°C. Die Gesamtausbeute betrug also 0,6 g (2,0 % d. Th.) (In der Litaratur wird ein Schmelzpunkt von 181° - 182°C angegeben).

**Beispiel 7:**

In diesem Beispiel wird eine Verbindung mit einem tertiären C-Atom, nämlich 1-phenyl-1-methoxy-ethan, mit dem Perketal 1,1-Bis(t-butylperoxy)-cyclohexan zum 2,3-Diphenyl-2,3-dimethoxy-butan analog Beispiel 1 dimerisiert:

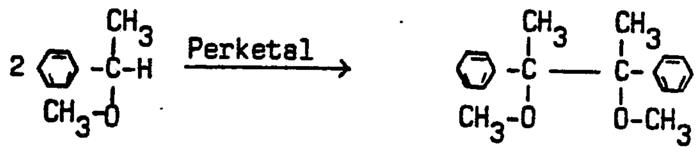


Abweichend von Beispiel 1 wurde das Perketal als 75,3 %-ige Lösung in n-Hexan eingesetzt; somit befanden sich vor Beginn im Rundkolben 36,3 g 1-Phenyl-1-methoxy-ethan (0,266 Mol) und im Tropftrichter eine Lösung von 38 g 1,1-Bis(t-butylperoxy)-cyclohexan (0,146 Mol) in 12,5 g n-Hexan und 90,5 g 1-Phenyl-1-methoxy-ethan (0,665 Mol). Bei der Ölbadtemperatur von 132°C lag die Innentemperatur bei 122°C und bei der erhöhten Ölbadtemperatur von 141°C lag die Innentemperatur bei 126°C. Nach dem Abdestillieren des unumgesetzten 1-Phenyl-1-methoxy-ethans i. Vak. bei etwa 120°C wurde der Rückstand über Nacht stehen gelassen. Die ausgefallenen Kristalle wurden abgesaugt und anschließend mit 5 ml Aceton ausgekocht. Nach dem Abkühlen und einigem Stehen wurden die Kristalle abgesaugt und getrocknet. Man erhielt so 4,4 g (11 % d. Th.) 2,3-Diphenyl-2,3-dimethoxy-butan als fast farbloses schwach gelbliches Kristallpulver vom Schmp. 152° - 164°C (Literatur: 170° - 172°C).

**Vergleichsbeispiel F:**

In diesem Beispiel wurde 1-Phenyl-1-methoxy-ethan wie in Beispiel 7 dehydrierend dimerisiert; anstelle des Perketals 1,1-Bis(t-butylperoxy)-cyclohexan wurden jedoch 0,146 Mol des nichterfindungsgemäßen Dialkylperoxids Di-t-butylperoxid eingesetzt. Auf diese Weise wurden nur 1,6 g (4,1 % d. Th.) 2,3-Diphenyl-2,3-dimethoxy-butan als fast farblose, schwach gelbliches Kristallpulver vom Schmp. 161° - 168°C (Literatur: 170° - 172°C) erhalten.

**Beispiel 8:**

In diesem Beispiel wird eine Verbindung mit einem tertiären C-Atom, nämlich sec.-Butyl-benzol, mit dem Perketal 1,1-Bis(t-butylperoxy)-cyclo-hexan zum 3,4-Diphenyl-3,4-dimethyl-hexan dimerisiert:

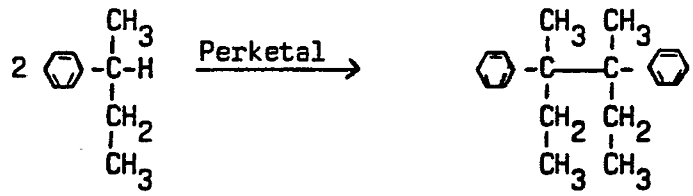


Die Herstellung des 3,4-Diphenyl-3,4-dimethyl-hexans erfolgt auf die gleiche Weise wie die des Biscumyls wie sie in Beispiel 1 beschrieben wurde, jedoch wird das Cumol durch sec.-Butyl-benzol ersetzt und zwar werden in den 250 ml-Dreihalsrundkolben 36 g sec.-Butyl-benzol (0,268 Mol) (anstelle von 32 g Cumol) eingewogen und die 38 g 1,1-Bis(t-butylperoxy)-cyclohexan (0,146 Mol) werden in 89 g sec.-Butyl-benzol (0,665 Mol) (anstelle von 80 g Cumol) gelöst. Außerdem wird nach beendeter Umsetzung die Entfernung des unumgesetzten sec.-Butyl-benzols und anderer flüchtiger Bestandteile im Wasserstrahlvakuum am Ende bei 130°C Ölbadtemperatur (anstelle von 100°C in Beispiel 1) durchgeführt. Nach beendeter Vakuumdestillation verbleibt im Kolben ein Rückstand bestehend aus 40,7 g eines hellgelbbraunen, sehr zähen Öls, das, wie durch HPLC-Analyse festgestellt wurde, 79 % 3,4-Diphenyl-3,4-dimethyl-hexan enthält, was einer Ausbeute von 83 % d. Th. entspricht.

**Beispiel 9:**

Typisches erfindungsgemäßes Beispiel zur Herstellung von Biscumyl (= 2,3-Diphenyl-2,3-dimethyl-butan) aus Cumol

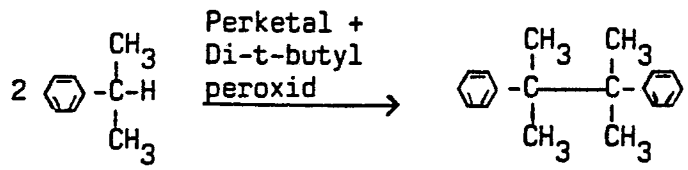


Mol-Gew. = 238,36
Schmp. (rein)= 119-120°C

Mol-Gew. = 238,36
Schmp. (rein) = 119 - 120°C
unter Verwendung des Perketals 2,2-Bis(t-butylperoxy)-butan und Di-t-butylperoxid

a) 32 g Cumol (= Isopropylbenzol) (0,266 Mol) wurden in einem 250- oder 500 ml-Dreihalsrundkolben eingewogen, der mit einem Rückflußkühler (am Mittelhals), einem absteigenden Kühler (an einem der zwei Außenhälse) und einem in das eingewogene Cumol hineinreichenden Thermometer (am zweiten der zwei Außenhälse) verbunden ist. Außerdem wurde durch den Rückflußkühler ein Glasrohr gesteckt, dessen unteres Ende unter die Oberfläche des Reaktionsgemisches reichte. Während des Aufheizens und der Umsetzung wurde mit einem Magnetrührer gerührt. Der Rundkolben befand sich in einem Ölbad. Man erwärmte das Ölbad auf 130°C und ließ - sobald das im Kolben befindliche Cumol eine Temperatur von etwa 125°C erreicht hatte - innerhalb 1 1/2 Stunden unter Rühren durch das durch den Rückflußkühler reichende, in das Cumol eintauchende Glasrohr eine Lösung von 34,2 g 2,2-Bis(t-butylperoxy)-butan (0,146 Mol) und 34,2 g Di-t-butylperoxid (0,234 Mol) in 114 g Cumol (0,948 Mol) zutropfen. Das Gewichtsverhältnis Perketal zu Di-t-butylperoxid beträgt also 1 : 1 und das Gew.-%-Verhältnis 50 % : 50 %. Während der Umsetzung destillieren die flüchtigen Zersetzungsprodukte der beiden Peroxide und ein Teil des Di-t-butylperoxids durch den absteigenden Kühler ab. Die Ölbadtemperatur wurde während der 3 Stunden Reaktionszeit in bestimmten Abständen erhöht. Die nachstehende Tabelle zeigt die jeweilige Ölbadtemperatur mit den dazugehörigen Zeiten und Innentemperaturen:

| | Zeiten | Ölbadtemp. | Innentemp. |
|---|---|---|---|
| Zutropfen. | 1 Stunde | 130°C | 125 - 126°C |
| " | 1/2 " | 137°C | 126,5 - 125°C |
| Reaktion: | 1/2 " | 145°C | 130,5 - 135°C |
| " | 1/2 " | 150°C | 140°C |
| " | 1/2 " | 155°C | 141,5 - 140,5°C |
| | 3 Stunden | | |

Die Gesamtreaktionszeit betrug also 3 Stunden. Nicht nur beim Zutropfen der Peroxid-Cumol-Lösung sondern auch während der Nachreaktion destillierte unverbrauchtes Di-t-butylperoxid gemeinsam mit den Zersetzungsprodukten der Peroxide, hauptsächlich t-Butanol, über. Im Destillat (32,6 g) waren 25,7 % (= 8,4 g) Di-t-butylperoxid, wie gaschromatographisch nachgewiesen wurde, enthalten. Danach wurde das überschüssige Cumol (Siedepunkt: 152° - 154°C) im Wasserstrahlvakuum bei maximal 100°C Ölbadtemperatur gemeinsam mit dem noch im Reaktionsgemisch vorliegenden Di- t-butylperoxid abdestilliert. Das Destillat wog 91,7 g und enthielt 7,1 % (= 6,5 g) Di-t-butylperoxid. Die Gesamtmenge an Di-t-butylperoxid in den Destillaten betrug also 14,9 g, das sind 43,6 Gew.-% der eingesetzten Menge Di-t-butylperoxid. Der Rückstand wurde aus 200 ml Isopropanol umkristallisiert und gab nach Absaugen und Trocknen an der Luft bei Raumtemperatur 40,4 g Biscumyl als weiße, kristalline Substanz vom Schmelzpunkt 114° - 117°C. Durch vollständiges Abdestillieren des Isopropanols aus dem Filtrat und Umkristallisieren des Rückstandes aus 10 ml Isopropanol wurden weitere 2,95 g weißes, kristallines Biscumyl vom Schmelzpunkt 103° - 107°C erhalten. Die Gesamtausbeute an Biscumyl liegt demnach bei 43,35 g (125 % d. Th. bezogen auf Perketal 2,2-Bis(t-butylperoxy)-butan allein). Sowohl das abdestillierte Cumol als auch das abdestillierte Isopropanol lassen sich wiederverwenden, auch das im "Reaktions"-Destillat befindliche Di-t-butylperoxid; dieses Destillat allerdings erst nach 2 - 3-maligem Waschen mit Wasser zur Entfernung des t-Butanols und der anderen wasserlöslichen Zersetzungsprodukte und Trocknen des Di- t-butylperoxids mit z. B. $Na_2SO_4$ oder $MgSO_4$.

b) Biscumyl wurde auf die gleiche Weise wie oben unter 9a) beschrieben aus 1,214 Mol Cumol (146 g) und 0,146 Mol des Perketals 2,2-Bis(t-butylperoxy)-butan (34,2 g), aber einer geringeren Menge Di-t-butylperoxid, nämlich 8,6 g (0,06 Mol) hergestellt. Hier betrug also das Gewichtsverhältnis Perketal zu Di-t-butylperoxid 4 : 1 und das Gew.-%-Verhältnis 80 % : 20 %. Die anschließende Tabelle zeigt die jeweiligen Ölbadtemperaturen mit den dazugehörigen Zeiten und Innentemperaturen:

| | Zeiten | Ölbadtemp. | Innentemp. |
|---|---|---|---|
| Zutropfen | 1 Stunde | 130°C | 125,5 - 128°C |
| " | 1/2 " | 137°C | 126,5 - 128,5°C |
| Reaktion | 1/2 " | 145°C | 138 - 141°C |
| " | 1/2 " | 150°C | 141 - 142,5°C |
| " | 1/2 " | 155°C | 143,5°C |
| | 3 Stunden | | |

Es wurden 31,3 g Biscumyl vom Schmelzpunkt 113,5 - 116,5°C und aus der Mutterlauge 3,3 g Biscumyl vom Schmp. 105 - 109°C, insgesamt also 34,6 g (100 % d. Th. bezogen auf Perketal 2,2-Bis(t-butylperoxy)-butan allein) Biscumyl in Form weißer Kristalle erhalten.

c) Biscumyl wurde auf die gleiche Weise wie oben unter 9a) beschrieben aus 1,214 Mol (146 g) und 0,146 Mol des Perketals 2,2-Bis(t-butylperoxy)-butan (34,2 g), aber einer geringeren Menge Di-t-butylperoxid, nämlich 11,4 g (0,08 Mol) hergestellt. Hier betrug also das Gewichtsverhältnis Perketal zu Di-t-butylperoxid 3 : 1 und das Gew.-%-Verhältnis 75 % : 25 %. Die Ölbadtemperatur und die dazugehörigen Zeiten und Innentemperaturen sind identisch mit denen aus dem Versuch 1a).

Im "Reaktions"-Destillat wurden gaschromatographisch 13,3 % (= 2,66 g) und im abdestillierten Cumol 28 % (= 2,49 g), insgesamt also 5,15 g, das sind 45,2 % des eingesetzten Di-t-butylperoxids, nachgewiesen. Es wurden 35,4 g Biscumyl vom Schmp. 114° - 117°C und aus der Mutterlauge 2,95 g Biscumyl vom Schmp. 104° - 110°C, insgesamt also 38,35 g (110 % d. Th. bezogen auf Perketal 2,2-Bis(t-butylperoxy)-butan allein) Biscumyl in Form weißer Kristalle erhalten.

d) Biscumyl wurde auf die gleiche Weise wie oben unter 9a) beschrieben aus 1,214 Mol Cumol (146 g) und 0,146 Mol des Perketals 2,2-Bis(t-butylperoxy)-butan (34,2 g), aber einer größeren Menge Di-t-butylperoxid, nämlich 51,3 g (0,351 Mol) hergestellt. Hier betrug also das Gewichtsverhältnis Perketal zu Di-t-butylperoxid 1 : 1,5 und das Gew.-%-Verhältnis 40 % : 60 %. Die unten stehende Tabelle zeigt die jeweiligen Ölbadtemperaturen mit den dazugehörigen Zeiten und Innentemperaturen:

| | Zeit | Ölbadtemp. | Innentemp. |
|---|---|---|---|
| Zutropfen: | 1 Stunde | 130°C | 124 - 126°C |
| " | 1/2 " | 137°C | 125 - 123°C |
| Reaktion: | 1/2 " | 145°C | 126 - 132°C |
| " | 1/2 " | 150°C | 135 - 140°C |
| " | 1/2 " | 155°C | 142 - 142,5°C |
| | 3 Stunden | | |

Im "Reaktions"-Destillat wurden gaschromatographisch 36 % (= 16,6 g) und im abdestillierten Cumol 11,3 % (= 9,85 g), insgesamt also 26,45 g, das sind 51,6 % des eingesetzten Di-t-butylperoxids, nachgewiesen. Es wurden 41,2 g Biscumyl vom Schmp. 115° - 120°C und aus der Mutterlauge 3,35 g Biscumyl vom Schmp. 105° - 110°C, insgesamt also 44,55 g (128 % d. Th. bezogen auf Perketal 2,2-Bis(t-butylperoxy)-butan allein) Biscumyl in Form weißer Kristalle erhalten.

**Beispiel 10** (Vergleich zu Beispiel 9):

Biscumyl wurde wie oben in Beispiel 9a) beschrieben aus 146 g Cumol (1,214 Mol), jedoch unter alleiniger Verwendung von 34,2 g des Perketals 2,2-Bis(t-butylperoxy)-butan (0,146 Mol) als Dehydrierungsmittel, also ohne Di-t-butylperoxid, hergestellt. Zutropfzeiten, Reaktionszeiten, Ölbadtemperaturen und Innentemperaturen sind aus nachstehender Tabelle zu ersehen:

| | Zeiten | Ölbadtemp. | Innentemp. |
|---|---|---|---|
| Zutropfen: | 1 1/2 Stunden | 140°C | 135 - 127°C |
| Reaktion: | 1/2 Stunde | 145°C | 139°C |
| " | 1/2 " | 150°C | 141°C |
| " | 1/2 Stunde | 155°C | 143°C |
| | 3 Stunden | | |

Die Gesamtreaktionszeit betrug also 3 Stunden. Es wurden 28 g Biscumyl vom Schmp. 115° - 117,5°C und aus der Mutterlauge 4,3 g Biscumyl vom Schmp. 109° - 114°C, insgesamt also 32,3 g (93 % d. Th.) Biscumyl in Form weißer Kristalle erhalten.

**Beispiel 11:**

a) In diesem erfindungsgemäßen Beispiel wurde auf die gleiche Weise wie in Beispiel 9a) Biscumyl aus 146 g Cumol (1,214 Mol) und 38 g Di-t-butylperoxid (0,26 Mol) hergestellt, jedoch unter Verwendung von 38 g des Perketals 1,1-Bis(t-butylperoxy)-cyclohexan (0,146 Mol) anstelle von 34,2 g 2,2-Bis(t-butylperoxy)-butan. Das Gew.-%-Verhältnis Perketal zu Di-t-butylperoxid betrug also 50 % : 50 %. Zutropfzeiten, Reaktionszeiten, Ölbadtemperaturen und Innentemperaturen sind aus nachstehender Tabelle zu ersehen:

| | Zeiten | Ölbadtemp. | Innentemp. |
|---|---|---|---|
| Zutropfen: | 1 Stunde | 130°C | 125 - 126°C |
| " | 1/2 " | 137°C | 127 - 126°C |
| Reaktion: | 1/2 " | 145°C | 131,5 - 135,5°C |
| " | 1/2 " | 150°C | 137 - 139,5°C |
| " | 1/2 " | 155°C | 140 - 142°C |
| | 3 Stunden | | |

Die Gesamtreaktionszeit betrug also 3 Stunden. Es wurden 26,3 g Biscumyl vom Schmp. 112° - 115,5°C und aus der Mutterlauge 7,5 g Biscumyl vom Schmp. 106° - 110°C, insgesamt also 33,8 g (97 % d. Th. bezogen auf Perketal 1,1-Bis(t-butylperoxy)-cyclohexan allein) Biscumyl in Form weißer Kristalle erhalten.

b) Biscumyl wurde auf die gleiche Weise wie oben unter 10a) beschrieben aus 146 g Cumol (1,214 Mol) und 38 g 1,1-Bis(t-butylperoxy)-cyclohexan (0,146 Mol), jedoch einer kleineren Menge Di-t-butylperoxid, nämlich 9,4 g (0,064 Mol) hergestellt. Hier betrug also das Gew.-%-Verhältnis

Perketal : Di-t-butylperoxid 80 % : 20 %. Die Gesamtreaktionszeit betrug wieder 3 Stunden. Es wurden 20,7 g Biscumyl vom Schmp. 112° - 115°C und aus der Mutterlauge 4,8 g Biscumyl vom Schmp. 110° - 112,5°C, insgesamt also 25,5 g (73,5 % d. Th. bezogen auf Perketal 1,1-Bis(t-butylperoxy)-cyclohexan allein) Biscumyl in Form weißer Kristalle erhalten.

c) Biscumyl wurde auf die gleiche Weise wie oben unter 10a) beschrieben aus 146 g Cumol (1,214 Mol) und 38 g 1,1-Bis(t-butylperoxy)-cyclohexan (0,146 Mol), jedoch einer größeren Menge Di-t-butylperoxid, nämlich 57 g (0,39 Mol) hergestellt. Hier betrug also das Gew.-%-Verhältnis Perketal zu Di-t-butylperoxid 40 % : 60 %. Die Gesamtreaktionszeit lag wieder bei 3 Stunden. Es wurden 30,6 g Biscumyl vom Schmp. 110° - 114°C und aus der Mutterlauge 7,3 g vom Schmp. 103° - 108°C, insgesamt also 37,9 g (109 % d. Th. bezogen auf Perketal 1,1-Bis(t-butylperoxy)-cyclohexan allein) Biscumyl in Form weißer Kristalle erhalten.

**Beispiel 12** (Vergleich zu Beispiel 11):

Biscumyl wurde wie oben in Beispiel 10a) beschrieben aus 112 g Cumol (0,931 Mol), jedoch unter alleiniger Verwendung von 38 g des Perketals 1,1-Bis(t-butylperoxy)-cyclohexan (0,146 Mol) als Dehydrierungsmittel, also ohne Di-t-butylperoxid hergestellt. Zutropfzeiten, Reaktionszeiten, Ölbadtemperaturen und Innentemperaturen sind aus nachstehender Tabelle zu ersehen:

| | Zeiten | Ölbadtemp. | Innentemp. |
|---|---|---|---|
| Zutropfen: | 1 1/2 Stunden | 140°C | 135 - 137°C |
| Reaktion: | 1/2 Stunde | 145°C | 140 - 141,5°C |
| " | 1/2 " | 150°C | 143,5°C |
| " | 1/2 " | 155°C | 148,5°C |
| | 3 Stunden | | |

Die Gesamtreaktionszeit betrug also 3 Stunden. Es wurden 20,1 g Biscumyl vom Schmp. 113 - 117°C und aus der Mutterlauge 4,3 g Biscumyl vom Schmp. 103°-111°C, insgesamt also 24,4 g (70 % d. Th.) Biscumyl in Form weißer Kristalle erhalten.

## Patentansprüche

1. Verfahren zur dehydrierenden Dimerisierung und/oder Polymerisierung von organischen Verbindungen mit beweglichem Wasserstoff an primären, sekundären oder tertiären C-Atomen, die bei Atmosphärendruck bei mindestens 95° C sieden und keine sauren Gruppen enthalten, unter Verwendung von Peroxiden als Dehydrierungsmittel und unter drucklosen Bedingungen bei Temperaturen von unterhalb bis knapp oberhalb des Siedepunktes der zu dehydrierenden Verbindungen, aber oberhalb der Zersetzungstemperatur der als Dehydrierungsmittel wirkenden Peroxide, in einer Vorrichtung, die ein beheizbares Reaktionsgefäß und einen damit verbundenen Rückflußkühler aufweist, dadurch gekennzeichnet, daß man

- als dehydrierende Peroxide ein oder mehrere Bis(alkylperoxy)-ketale und/oder Bis(aralkylperoxy)ketale mit 10 Stunden-Halbwertszeit-Temperaturen von weniger als 115°C (bestimmt in Benzol) oder eine Kombination aus einem oder mehreren dieser Perketale mit Di-t-butylperoxid verwendet, und
- die flüchtigen Peroxidzersetzungsprodukte und gegebenenfalls einen Teil des Di-t-butylperoxids durch einen, zusätzlich zum Rückflußkühler vorhandenen, absteigenden Kühler abdestilliert.

2. Verfahren nach Anspruch 1, dadurch gekennzeichnet, daß dem absteigenden Kühler eine Destillationskolonne vorgeschaltet ist.

3. Verfahren nach Anspruch 1 oder 2, dadurch gekennzeichnet, daß es in Gegenwart von gegen den Angriff von Peroxidradikalen inerten, unter 120°C siedenden Lösungsmitteln, die keinen beweglichen Wasserstoff aufweisen und vorzugsweise nur $CH_3$- und $CH_2$-Gruppen enthalten, erfolgt.

4. Verfahren nach einem der Ansprüche 1 bis 3, dadurch gekennzeichnet, dass es bei Temperaturen zwischen 100 und 180°C durchgeführt wird.

5. Verfahren nach einem der Anprüche 1 bis 4, dadurch gekennzeichnet, daß es bei wenigstens 120°, vorzugsweise bei Temperaturen zwischen 130 und 155°C, durchgeführt wird.

## Claims

1. A process for dehydrogenating dimerization and/or polymerization of organic compounds with mobile hydrogen at primary, secondary, or tertiary carbon atoms which, at atmospheric pressure, boil at least at 95°C and which do not contain any acidic groups, with the use of peroxides as dehydrogenating agents and under unpressurized conditions at temperatures from below up to barely above the boiling point of the compounds to be dehydrogenated, but above the decomposition temperature of the peroxides serving as dehydrogenating agents, in an apparatus comprising a heatable reactor and a reflux condenser connected thereto, characterized by using

- one or more bis-(alkylperoxy)-ketals and/or bis(aralkylperoxy)-ketals with 10 hours' half-life temperatures of less than 115°C (determined in benzene) or a combination of one or more of said perketals with di-t-butyl peroxide, and
- distilling off the volatile peroxide decomposition products and optionally a portion of the di-t-butyl peroxide by a descending condenser provided in addition to the reflux condenser.

2. Process according to claim 1, characterized in that upstream of the descending condenser a distillation column is provided.

3. Process according to claim 1 or 2, characterized in that it takes place in the presence of solvents inert toward attack by peroxide radicals, boiling below 120°C, containing no mobile hydrogen, and preferably containing only $CH_3$ and $CH_2$ groups.

4. Process according to one of claims 1 to 3, characterized in that it is carried out at temperatures between 100 and 180°C.

5. Process according to one of claims 1 to 4, characterized in that it is carried out at least at 120°C, preferably at temperatures between 130 and 155°C.

## Revendications

1. Procédé de dimérisation et/ou de polymérisation par déshydrogénation de composés organiques contenant de l'hydrogène mobile sur des atomes primaires, secondaires ou tertiaires de carbone, qui bouillent à la pression atmosphérique à au moins 95°C et qui ne contiennent pas de groupes acides, en utilisant des peroxydes comme agents déshydrogénants et dans des conditions n'impliquant pas de pression, à des températures allant d'une valeur inférieure à une valeur juste supérieure au point d'ébullition des composés à déshydrogéner, mais au-dessus de la température de décomposition des peroxydes agissant comme agents déshydrogénants, dans un dispositif qui présente un réacteur pouvant être chauffé et un réfrigérant à reflux relié à ce réacteur, caractérisé en ce que:

- on utilise comme peroxydes déshydrogénants un ou plusieurs bis(alkylperoxy)-cétals et/ou bis(aralkylperoxy)-cétals ayant des températures, pour un temps de demi-réaction de 10 heures, de moins de 115°C (comme déterminé dans le benzène) ou une association d'un ou plusieurs de ces percétals avec du peroxyde de ditertio-butyle, et
- on chasse par distillation les produits volatils de décomposition des peroxydes et le cas échéant une partie du peroxyde de di-tertio-butyle par un réfrigérant descendant prévu en plus du réfrigérant à reflux.

2. Procédé suivant la revendication 1, caractérisé en ce qu'une colonne de distillation est placée en amont du réfrigérant descendant.

3. Procédé suivant la revendication 1 ou 2, caractérisé en ce qu'il est mis en oeuvre en présence de solvants bouillant au-dessous de 120°C, inertes vis-à-vis de l'attaque par des radicaux peroxydiques, qui ne présentent pas d'hydrogène mobile et qui ne contiennent de préférence que des groupes $CH_3$ et $CH_2$.

4. Procédé suivant l'une des revendications 1 à 3, caractérisé en ce qu'il est mis en oeuvre à des températures comprises entre 100 et 180°C.

5. Procédé suivant l'une des revendications 1 à 4, caractérisé en ce qu'il est mis en oeuvre à une température d'au moins 120°C, de préférence à des températures comprises entre 130 et 155°C.